# EUROPEAN PATENT SPECIFICATION

(11) **EP 2 381 874 B1**
(45) Date of publication and mention of the grant of the patent: **17.08.2016**
(21) Application number: 10702774.0
(22) Date of filing: 20.01.2010
(51) Int. Cl.: A61B 18/00, A61B 5/00, A61B 18/14

(54) **APPARATUS FOR MINIMIZING THERMAL TRAUMA TO AN ORGAN DURING TISSUE ABLATION OF A DIFFERENT ORGAN**
VORRICHTUNG ZUR MINIMIERUNG VON HITZETRAUMATA EINES ORGANS WÄHREND DER GEWEBEABLATION EINES ANDEREN ORGANS
APPAREIL PERMETTANT DE MINIMISER LE TRAUMATISME THERMIQUE D'UN ORGANE DURANT UNE ABLATION DE TISSU D'UN ORGANE DIFFÉRENT

(30) Priority: 20.01.2009 US 145800 P
(43) Date of publication of application: 02.11.2011
(73) Proprietor: Advanced Cardiac Therapeutics Inc., Laguna Beach, CA 92651 (US)
(72) Inventor: LENIHAN, Timothy, J., 50009 Hradee Kralove (CZ)
(74) Representative: Rupprecht, Kay
(86) International application number: PCT/US2010/000128
(87) International publication number: WO 2010/090701

(56) References cited:
- WO-A1-99/03535
- US-A- 5 354 325
- US-A- 5 992 419
- US-A1- 2007 055 328
- US-B1- 6 477 426

## Description

### BACKGROUND OF THE INVENTION

This invention relates to an apparatus for minimizing thermal injury to the esophagus during a cardiac ablation procedure. Anatomically, the esophagus is very close to, and often in contact with, part of the left atrium. Thus, ablating certain regions of the left atrium to treat various arrhythmias in the heart can unintentionally cause thermal damage to the esophagus, often with severe consequences. The present invention relates especially to a technique for measuring and monitoring the temperature of the esophagus wall at depth so as to avoid overheating that wall during cardiac ablation.

During a typical cardiac ablation procedure, an electrode catheter is used to resistively heat heart tissue, usually at the left side of the heart, sufficiently to intentionally damage the target tissue in order to cure a potentially fatal heart arrhythmia. Typically, heating the tissue to a temperature in excess of 70°C for 30-60 seconds is sufficient to cause necrosis. This procedure was first attempted over twenty years ago and has become the standard treatment method for most supraventricular tacchycardias (SVTs). During treatment, electromagnetic energy, usually in the RF frequency range, is applied between the tip of the electrode catheter and a ground plate removably affixed to the patient's back, creating an electrical circuit. The point of highest resistance in this circuit, normally the interface between the catheter tip and the heart tissue, is the region which heats the most and thus may cause intentional, irreversible damage to the heart tissue to correct the arrhythmia.

In a standard SVT ablation procedure, the heat generated in the tissue contacted by the catheter is monitored with a temperature sensor such as a thermistor or a thermocouple in the catheter tip. A signal from the sensor is applied to a display in an external control unit, enabling the operating surgeon to adjust the power to the ablation catheter as needed to provide sufficient heating of the tissue to cause necrosis, but not enough to result in surface charring of the tissue that could cause a stroke and/or the formation of microbubbles (popping) that could rupture the heart vessel wall. The same output from the temperature sensor is also sometimes used to provide a feedback signal to the RF generator to automatically control heating of the tissue contacted by the ablation catheter.

With experience over time, surgeons have found a need to burn tissue on the left side of the heart increasingly deeper to achieve a favorable patient outcome. In order to minimize the above-mentioned surface charring of the tissue, the tips of today's ablation catheter may be cooled by a circulating a fluid through the catheters. However, with this artificial cooling came much deeper lesions and, due to the relatively close position of the esophagus to a region of the left atrium which is often ablated during such procedures, there is a great risk that ablating parts of the left atrium which are intended to be heated and thus destroyed, could inadvertently overheat and injure the esophagus. This can lead to serious complications, such as ulcers of the esophagus, bleeding, perforation of the esophagus wall and even the death of the patient.

There do exist catheter apparatus for insertion into the esophagus during a cardiac ablation procedure that are intended to prevent thermal damage to the esophagus. One such apparatus delivers cooled fluid through a balloon catheter to the esophagus wall, employing a heat exchange principle to lower the temperature of that wall; see e.g. US 2007/0055328 A1. Another type of apparatus uses a catheter carrying conventional point source temperature sensors such as thermocouples, thermistors, fiberoptic probes or the like to monitor, and ultimately prevent the overheating of, the esophagus wall by cutting off or reducing the power delivered to the ablation catheter; see e.g. US 2007/0066968 A1.

In the case of the former type esophageal catheter which only cools the esophagus, even with constant irrigation of the inner surface of the esophagus, damage can still occur in the wall or on the outer surface of the esophagus, and in this type of instrument, there is no way to know if effective cooling of the wall of the esophagus is being achieved. That is, as with many active cooling catheters, e.g. an RF ablation catheter, once a coolant is introduced, no conventional temperature sensors can be used to monitor tissue temperature because they only sense temperature at a point and not at depth. Therefore, they only measure the temperature of the coolant and not of the tissue. Thus, even if such esophageal cooling catheters should allow for temperature measurement, they would not be able to measure accurately esophageal temperature once cooling is initiated. Moreover, while surface cooling can be achieved with these catheters, there is no indication of the effectiveness of the cooling and there is no measurement of temperature rises at depth in the wall or at the outer surface of the esophagus.

The latter type esophageal catheter above, which has conventional temperature sensors on the outer surface thereof, is only capable of measuring the temperature of the inner surface of the esophagus and because it can only measure at a point and not at depth, it provides a very late indication of problems with overheating of the esophagus.

In clinical cases, using conventional surface sensors, surgeons have reported thermal damage to the esophagus only after a temperature rise of 1-2°C is recorded. This is because there is clearly heat buildup deep in the esophageal wall which is not detected or recorded by such catheters.

US 5,992,419 teaches an apparatus without passages connected to small holes.

### SUMMARY OF THE INVENTION

Accordingly, it is an object of this application to provide a method for accurately measuring esophageal wall temperature at depth whether or not the esophagus is being cooled.

Another object of the application is to provide a method for effectively cooling the inner surface of the esophagus during an ablation procedure in order to protect the esophagus from unintended thermal damage while accurately measuring the temperature at depth and at the outer surface of the esophageal wall. A further object of the application is to provide a method for accurately measuring the effectiveness of the overall cooling not only of the inner surface of the esophagus, but also deep in the esophagus wall and at the outer surface thereof.

Yet another object of the application is to provide such a method which minimizes the chances of causing a perforated esophagus or an atrioesophageal fistula (i.e. unwanted connection between the left atrium and the esophagus).

A further object of the application is to provide a method of this type which maximizes the information provided to an operating surgeon to prevent unintended damage to tissue during an ablation procedure.

Still another object of the application is to provide such a method which can provide an indication that the outer wall of the heart adjacent to the esophagus has been successfully ablated before damage to the esophagus can occur.

A further object of the application is to provide a method of this type which facilitates measuring a temperature coming from a given direction.

An additional object is to provide such a method which facilitates a temperature measurement coming from all directions (omni-directional).

An object of the invention is to provide apparatus for implementing the above method.

Yet another object of the invention is to provide apparatus for measuring esophageal temperature during cardiac ablation which improves the chances of a favorable patient outcome.

A further object of the invention is to provide apparatus for measuring esophageal temperature which can provide a control signal to associated apparatus to prevent unintended tissue damage.

Other objects will, in part, be obvious and will, in part, appear hereinafter. The invention accordingly comprises the several steps and the relation of one or more of such steps with respect to each of the others, and the apparatus embodying the features of construction, combination of elements and arrangement of parts which are adapted to effect such steps, all as exemplified in the following detailed description, and the scope of the invention will be indicated in the claims.

Briefly, in accordance with this method, a temperature sensing microwave antenna probe is inserted into a body passage or cavity that is adjacent to the tissue to be ablated so that the probe is on the other side of the passage or cavity wall from that tissue. We will describe the method as practiced during a cardiac ablation procedure in which the probe is placed in a patient's esophagus next to the heart. However, it should be understood that the method could be used in connection with other procedures such as the treatment of benign prosthetic hyperplasia (BPH) in which an ablation catheter is positioned in the patient's urethra and the temperature probe incorporating this invention is located in the rectum.

Obviously, in order to perform its function, the temperature probe must be small in diameter and quite flexible so that it can be threaded into the body passage to the target site. The probe may also be required to facilitate various ancillary processes using known means such as display of the target site, irrigation or cooling of the target site, etc.

The temperature probe is connected by a long, flexible service line to an external control unit which includes a receiver, preferably in the form of a radiometer, which detects the microwave emissions picked up by the antenna in the probe which emissions reflect the temperature of the tissue being examined. The receiver thereupon produces a corresponding temperature signal which may be used to control a display to indicate that temperature.

Preferably, the probe antenna is impedance matched to a selected frequency range enabling it to pick up emissions from relatively deep regions in the wall of the passage or cavity in which it is placed and even from the outer surface of that wall.

During a cardiac ablation procedure prescribed for cardiac arrhythmia, an ablation catheter is threaded into the left atrium of the heart such that energy can pass from the catheter tip into the tissues of the posterior wall of the left atrium. Heating at that wall then occurs, leading to localized necrosis of the left atrium creating a lesion which stops the arrhythmia.

In accordance with this method, during such a procedure, the temperature at depth in the esophageal tissue which is in close proximity to the ablation site in the patient's heart is measured using microwave radiometry and that measurement is used to determine the potential damage which could be caused to the esophagus unintentionally. Because microwave radiometry measures a volumetric temperature, that measurement is independent of the angle of contact of the temperature probe to the tissue, unlike the case of conventional temperature-sensing catheters utilizing thermistors and thermocouples which only measure a point on the tissue. Also due to the nature of microwave radiometry, the temperature at depth in the wall of the esophagus can be measured accurately even when the esophagus is being cooled.

Thus, using this method and apparatus, a surgeon may observe in real time esophageal temperature while tissue is being ablated in the left side of the heart. When the energy from the cardiac ablation catheter starts to heat beyond the outer wall of the heart and inadvertently starts to heat the adjacent anterior surface of the esophagus, there is a noticeable temperature rise picked up by the temperature probe situated in the esophagus so that the apparatus' display provides the surgeon with a clear, early warning of potential damage to the esophagus. This is very important given the severe consequence of any damage to the esophagus as discussed above.

As also noted above, due to the nature of microwave radiometry, the temperature probe used to practice my method may include a cooling function to cool the esophagus wall while still accurately monitoring the wall tissue temperature.

In addition, using this probe, a surgeon can even indirectly monitor the temperature of the outer surface of the heart opposite the esophagus to determine if the heart outer wall is sufficiently ablated which is a great indicator of success for treatment of such diseases as atrial fibrillation.

Unlike the case with conventional temperature probing techniques, the present method and apparatus which facilitate the safe ablation of the heart while avoiding inadvertent overheating of the esophagus are essentially independent of the surface temperature of the probe itself due to artificial cooling. This is because microwave radiometry measures tissue temperature at depth and is a function of the antenna pattern produced by the antenna in the probe.

Finally, the temperature signals from the temperature probe may be used to control the cooling of the temperature probe if the probe includes a cooling function. Those same signals may also be used to help control the power delivered to an associated ablation catheter that is being used to ablate the heart tissue.

### BRIEF DESCRIPTION OF THE DRAWINGS

For a fuller understanding of the invention, reference should be made to the following detailed description taken in connection with the accompanying drawings, in which:
FIG. 1 is a diagrammatic view of a patient's head and torso showing an ablation catheter in the left atrium of the heart and a temperature probe with a microwave antenna according to this invention situated in the esophagus adjacent to the catheter;
FIG. 2 is a block diagram of apparatus for minimizing thermal damage to the esophagus during cardiac ablation that includes the FIG. 1 temperature probe;
FIG. 3 is a fragmentary side elevational view on a larger scale showing the FIG. 1 temperature probe in greater detail;
FIG. 4 is a diagrammatic view showing the antenna pattern of such a probe, and
FIG. 5 is a graphical representation showing the output of a radiometer measuring the temperature at depth during ablation of tissue using the temperature probe shown in FIG. 4.

### DESCRIPTION OF A PREFERRED EMBODIMENT

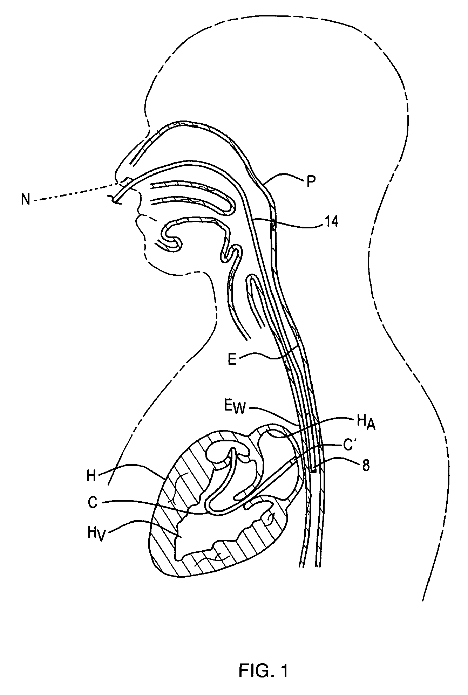

Refer first to FIG. 1 of the drawings which shows the head and torso of a patient having a heart H with a left ventricle H_{V} and a left atrium H_{A}. As is usually the case, the left atrium of the heart is very close to, if not in contact with, the anterior wall of the patient's esophagus E. During a cardiac ablation procedure, an ablation catheter C is threaded into the left atrium H_{A} via the left ventricle H_{V} so that the working end C' of the catheter contacts the posterior wall of the left atrium.

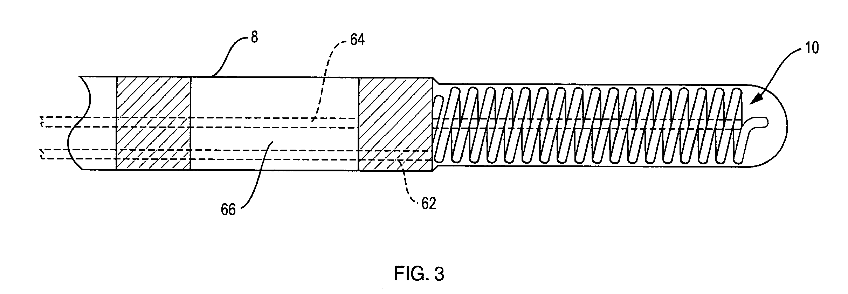

In order to prevent overheating of the esophagus E during such an ablation procedure, a temperature probe shown generally at 8 and containing a microwave antenna 10 (FIG. 3) may be inserted into the patient's nasal passage N and threaded down into the esophagus E via the patient's pharynx P until the probe is positioned directly opposite the catheter end C' at the ablation site as shown in FIG. 1. In accordance with this invention, as the heart H is being ablated by catheter C, the probe antenna 10 picks up microwave emissions from regions relatively deep in the esophageal wall E_{W} and produces corresponding temperature signals which may be used in a manner to be described presently to prevent overheating of the esophagus.

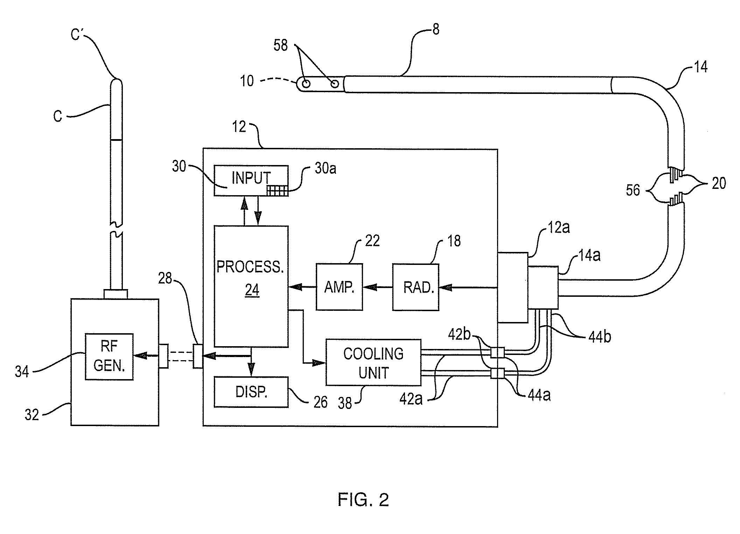

As shown in FIG. 2 of the drawings, the probe 8 may be connected to an external control unit 12 by way of a long, flexible service line 14 having an end connector 14a that connects to a mating connector 12a on unit 12. Typically, probe 8 may be in the order of 80-130 cm long and 1-10 mm in diameter and be steerable or nonsteerable.

The control unit 12 includes a radiometer 18 having an input to which the antenna 10 is connected by way of a coaxial cable 20 in service line 14. The radiometer produces a temperature signal corresponding to the microwave energy picked up by the antenna. The radiometer operates at a center frequency of 1 to 4 GHz, preferably 4 GHz, so that the apparatus can detect emissions from relatively deep regions of the esophagus wall, while not seeing too deep.

An amplifier 22 conditions the signal from the radiometer and routes it to a processor 24 which produces a corresponding control signal for controlling a display 26 which can display the temperature of the tissue being probed by the probe 8. Of course, the display 26 may also display other parameters relating to proper operation of the apparatus and preferably displays esophageal tissue temperature as a function of time so that the surgeon can see that temperature in real time. The processor 24 may also deliver the temperature signal to an output terminal 28 of unit 12. The processor 24 may receive instructions via the control buttons 30a of an operator-controlled input keyboard 30 on unit 12.

In certain applications, the control signal at terminal 28 may be coupled to an associated cardiac ablation apparatus 32 containing a RF generator 34 that powers the ablation catheter C. In this way, that control signal may be used to control the energy being delivered by the ablation catheter C to the target tissue in the heart H (FIG. 1).

As shown in FIG. 2, control unit 12 may also include a cooling unit 38 controlled by processor 24 and connected via one or more hoses 42a to corresponding connectors 42b on the outside of unit 12. Connectors 42b may be coupled to mating connectors 44a at the ends of conduits 44b leading to connector 14a. In connector 14a, the tubes 44b connect to one or more passages 56 in service line 14 so that a cooling fluid may be circulated to, and perhaps also from, probe 10. In the event that the cooling fluid is being used to irrigate esophagus E, small holes 58 connected to the passage(s) 56 may be provided in the catheter as shown in FIG. 2. The processor 24 may control the cooling unit 38 to increase or decrease the coolant flow rate to probe 8 and/or vary the coolant temperature to keep the portions of the esophagus wall E_{W} opposite catheter tip C' (FIG. 1) at a desired temperature.

Refer now to FIG. 3 which shows the antenna 10 in temperature probe 8 in greater detail. As seen there, the antenna is a helical antenna including an outer conductor 62, an inner conductor 64 and dielectric material 66, e.g. PTFE, having a low dielectric constant and low loss tangent, separating the two conductors. The proximal ends of the two conductors connect to the coaxial cable 20 in service line 14 and the dielectric material 66 may form fluid passages (not shown) leading from passage(s) 56 in line 14 to the holes 58 in probe 8. The antenna 10 may be of the type disclosed in U.S. Patent 5,683,382. The FIG. 3 antenna is axially symmetric and has an omnidirectional antenna pattern. However, as we shall see, the probe 8 could just as well contain a directional antenna which "looks" in a preferred direction, e.g. in the direction of heart H in FIG. 1. In either event, antenna 10 should be designed so that it provides a good impedance match to the selected radiometer frequency, e.g. 4 GHz.

When a cardiac ablation procedure is being performed by the associated ablation apparatus 32, the temperature probe 8 may be positioned in esophagus E opposite the catheter tip C' as shown in FIG. 1 and the monitoring apparatus used to sense the temperature at depth in the esophageal wall E_{W}. The temperature-indicating signals from the radiometer 18 are processed by processor 24 and display 26 displays the esophageal tissue temperature at the work site as a function of time. Thus, the operating surgeon can see that temperature in real time and react quickly to prevent the esophagus from being overheated by the ablation catheter C. For example, using the keypad 30, the surgeon may appropriately cool down probe 8 and/or reduce the power to ablation catheter C.

### Working Example:

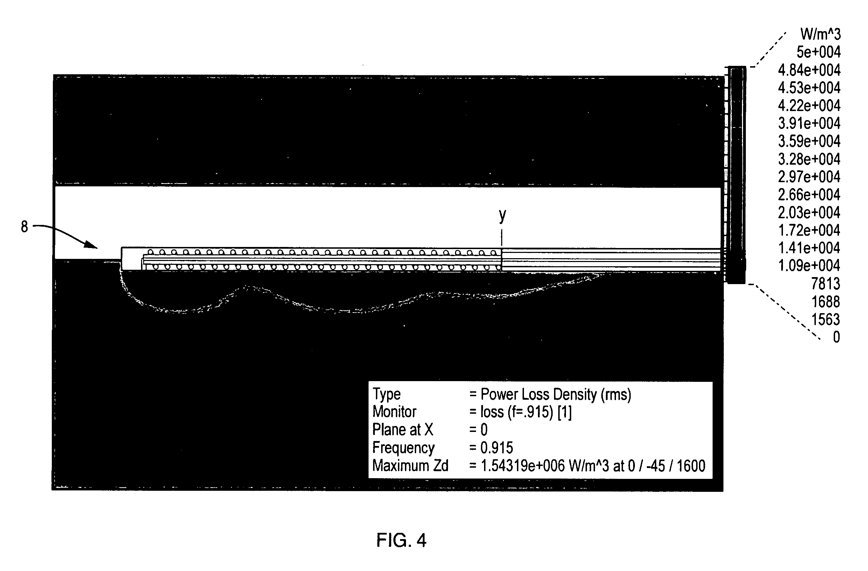

A test was performed using the temperature probe 8 depicted in FIG. 4 to verify that the temperature at depth in tissue can be recorded while part of the tissue is being cooled. Testing was done with the delivery of microwave power at 2.4 GHz via a catheter C to tissue which was actively cooled by body temperature saline solution running under the tissue to simulate blood flow and the probe 8 was positioned to record the temperature at depth in the tissue. The probe 8 in FIG. 4 is similar to probe 8 in FIG. 3 except that it has a body of low dielectric material above the antenna which causes the antenna to "look" down into the tissue as seen from the longitudinal sectional view of the antenna pattern in FIG. 4, i.e. the antenna is
directional. The antenna in probe 8 operates at a frequency of 4 GHz. It should be noted that the antenna pattern in FIG. 4 was obtained with the antenna in the transmit or radiate mode rather than the receive mode because this is the usual custom since reciprocity dictates that the two patterns are identical. In any event, it is apparent from FIG. 4 that the antenna pattern at the selected frequency is relatively uniform along the probe and reaches well into the tissue located below the probe.

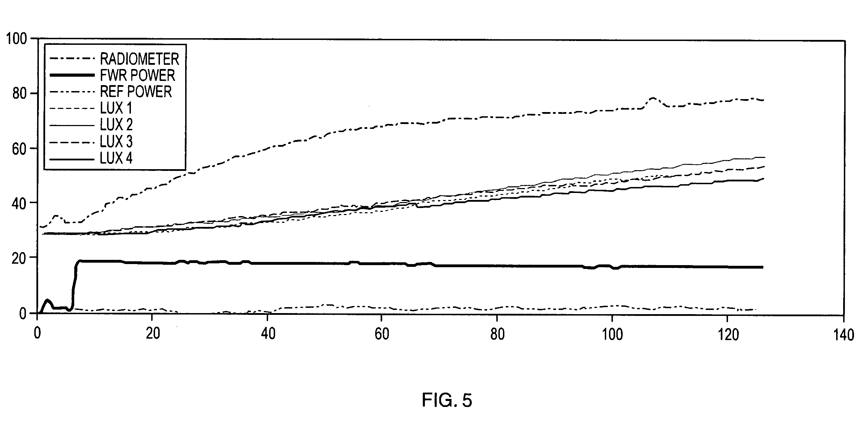

FIG. 5 graphs a typical test run wherein power was delivered to the tissue while the tissue was being cooled and with the probe 8 in FIG. 4 sensing temperature at depth in the tissue. As shown in FIG. 5, the radiometer reading indicated a temperature increase even while the tissue was being cooled.

In the Working Example, the temperature probe 8 could be inserted into a patient, i.e. into the esophagus E close to the left atrium of the heart. While observing the temperature reading on the display, the surgeon may alter the power delivered to the ablation catheter, shut off that power and/or increase the cooling effect on the temperature probe 8 by increasing the flow rate and/or temperature of the coolant delivered to that probe.

It will thus be seen that the objects set forth above, among those made apparent from the preceding description, are efficiently attained and, since certain changes may be made in carrying out the above method and in the constructions set forth without departing from the scope of the invention, it is intended that all matter contained in the above description or shown in the accompanying drawings shall be interpreted as illustrative and not in a limiting sense.

It is also to be understood that the following claims are intended to cover all of the generic and specific features of the invention described herein.

## Claims

1. Apparatus for minimizing thermal trauma during tissue ablation comprising:
an ablation catheter (C) for positioning at an ablation site on a first organ (H) in a patient's body;
a device (32) for providing energy to the ablation catheter to heat first organ tissue at the ablation site;
microwave radiometry apparatus including a probe (8) containing a microwave antenna (10) and a radiometer (18) responsive to the antenna output for producing a temperature signal corresponding to the thermal radiation picked up by the antenna (10), said probe being positionable in a
body passage of a second organ (E) in the patient's body having a wall portion adjacent to the ablation site so that the microwave antenna (10) is locatable at a measurement site opposite the ablation site, said radiometry apparatus being adapted to measure the temperature at depth in the second organ tissue at the measurement site to provide a corresponding temperature signal, an external control unit (12) controlling the ablation catheter (C) in response to the temperature signal to maintain said temperature of the second organ tissue below a predetermined value that does not result in thermal trauma to the second organ tissue; and
an apparatus (38) for flowing a cooling fluid through the probe (8), and a control device (24) for controlling the flow rate and/or temperature of the cooling fluid so as to cool the second organ tissue, wherein the probe (8) is connected to the external control unit (12) by way of a service line (14) having an end connector (14a) that connects to a mating connector (12a) on the control unit (12), the service line (14) including one or more passages (56) which are connected to small holes (58) in the probe (8).

2. The apparatus defined in claim 1 wherein the first organ is the heart and the second organ is the esophagus.

3. The apparatus defined in claim 1 wherein the first organ is the urethra and the second organ is the rectum.

4. The apparatus defined in claim 1 wherein the ablation catheter includes a heating element and the controller includes a device for regulating the current applied to the heating element.

5. The apparatus defined in claim 1 wherein the ablation catheter includes an RF antenna and the controller includes a device for regulating the energy supplied to the RF antenna.

6. The apparatus defined in claim 1 wherein the radiometry apparatus also includes a temperature indicator responsive to the temperature signal for indicating the temperature of the second organ tissue.

## Patentansprüche

1. Vorrichtung zur Minimierung von Hitzetraumata während einer Gewebeablation, Folgendes aufweisend:
einen Ablationskatheter (C) zur Positionierung an einer Ablationsstelle an einem ersten Organ (H) in einem Patientenkörper;
eine Vorrichtung (32), um dem Ablationskatheter Energie zum Erhitzen eines ersten Organgewebes an der Ablationsstelle bereitzustellen;
eine Mikrowellenradiometrievorrichtung mit einer Sonde (8), die eine Mikrowellenantenne (10) und ein Radiometer (18) enthält, das auf den Antennenausgang anspricht, um ein Temperatursignal zu erzeugen, das der von der Antenne (10) aufgenommenen Wärmestrahlung entspricht, wobei die Sonde in einem Körperdurchgang eines zweiten Organs (E) im Patientenkörper mit einem Wandbereich angrenzend an die Ablationsstelle so positionierbar ist, dass die Mikrowellenantenne (10) an einer Messstelle gegenüber der Ablationsstelle befindlich angeordnet werden kann, wobei die Radiometrievorrichtung dazu angepasst ist, die Temperatur in einer Tiefe im zweiten Organgewebe an der Messstelle zu messen, um ein entsprechendes Temperatursignal bereitzustellen,
eine externe Steuereinheit (12), die den Ablationskatheter (C) im Ansprechen auf das Temperatursignal steuert, um die Temperatur des zweiten Organgewebes unter einem vorbestimmten Wert zu halten, der nicht zu Wärmetraumata des zweiten Organgewebes führt; und
eine Vorrichtung (38), um ein Kühlfluid durch die Sonde (8) fließen zu lassen, und eine Steuervorrichtung (24) um den Durchsatz und/oder die Temperatur des Kühlfluids zu steuern, um das zweite Organgewebe zu kühlen, wobei die Sonde mittels einer Versorgungsleitung (14) mit einem Endsteckverbinder (14a) an die externe Steuereinheit (12) angeschlossen ist, der eine Verbindung zu einem Gegensteckverbinder (12a) an der Steuereinheit (12) herstellt, wobei die Versorgungsleitung (14) einen Durchgang oder mehrere Durchgänge (56) hat, die mit kleinen Öffnungen (58) in der Sonde (8) verbunden sind.

2. Vorrichtung nach Anspruch 1, wobei es sich bei dem ersten Organ um das Herz und dem zweiten Organ um den Ösophagus handelt.

3. Vorrichtung nach Anspruch 1, wobei es sich bei dem ersten Organ um die Urethra und dem zweiten Organ um das Rektum handelt.

4. Vorrichtung nach Anspruch 1, wobei der Ablationskatheter ein Heizelement umfasst und die Steuerung eine Vorrichtung zum Regeln des an das Heizelement angelegten Stroms umfasst.

5. Vorrichtung nach Anspruch 1, wobei der Ablationskatheter eine HF-Antenne umfasst und die Steuerung eine Vorrichtung zum Regeln der an die HF-Antenne gelieferten Energie umfasst.

6. Vorrichtung nach Anspruch 1, wobei die Radiometrievorrichtung auch eine Temperaturanzeige umfasst, die auf das Temperatursignal anspricht, um die Temperatur des zweiten Organgewebes anzuzeigen.

## Revendications

1. Appareil pour minimiser le traumatisme thermique pendant une ablation des tissus, comprenant :
un cathéter d'ablation (C) destiné à être positionné à un site d'ablation sur un premier organe (H) dans le corps d'un patient ;
un dispositif (32) pour fournir de l'énergie au cathéter d'ablation afin de chauffer un premier tissu d'organe au site d'ablation ;
un appareil de radiométrie à micro-ondes incluant une sonde (8) contenant une antenne à micro-ondes (10) et un radiomètre (11) réagissant à la sortie de l'antenne pour produire un signal de température correspondant au rayonnement thermique capté par l'antenne (10), ladite sonde étant susceptible d'être positionnée dans un passage corporel d'un second organe (E) dans le corps du patient, ayant une portion de paroi adjacente au site d'ablation, de sorte que l'antenne à micro-ondes (10) est capable d'être placée à un site de mesure à l'opposé du site d'ablation, ledit appareil de radiométrie étant adapté à mesurer la température en profondeur dans un second tissu d'organe au niveau du site de mesure pour fournir un signal de température correspondant,
une unité de commande externe (12) qui commande le cathéter d'ablation (C) en réponse au signal de température pour maintenir ladite température du second tissu d'organe au-dessous d'une valeur prédéterminée qui n'a pas pour résultat un traumatisme thermique au second tissu d'organe ; et
un appareil (3 8) pour amener un fluide de refroidissement à s'écouler à travers la sonde (8), et un dispositif de commande (24) pour commander le débit et/ou la température du fluide de refroidissement de manière à refroidir le second tissu d'organe, dans lequel la sonde (8) est connectée à l'unité de commande externe (12) au moyen d'une ligne de service (14) ayant un connecteur terminal (14a) qui est connecté à un connecteur apparié (12a) sur l'unité de commande (12), la ligne de service (14) incluant un ou plusieurs passages (56) qui sont connectés à des petits trous (58) dans la sonde (8).

2. Appareil selon la revendication 1, dans lequel le premier organe est le coeur et le second organe est l'oesophage.

3. Appareil selon la revendication 1, dans lequel le premier organe est l'urètre et le second organe est le rectum.

4. Appareil selon la revendication 1, dans lequel le cathéter d'ablation inclut un élément chauffant et le contrôleur inclut un dispositif pour réguler le courant appliqué à l'élément chauffant.

5. Appareil selon la revendication 1, dans lequel le cathéter d'ablation inclut une antenne à radiofréquences et le contrôleur inclut un dispositif pour réguler l'énergie fournie à l'antenne à radiofréquences.

6. Appareil selon la revendication 1, dans lequel l'appareil de radiométrie inclut également un indicateur de température réagissant au signal de température pour indiquer la température du tissu du second organe.
